# EUROPÄISCHE PATENTSCHRIFT

(11) **EP 3 709 957 B1**
(45) Veröffentlichungstag und Bekanntmachung des Hinweises auf die Patenterteilung: **01.09.2021**
(21) Anmeldenummer: 18830711.0
(22) Anmeldetag: 16.11.2018
(51) Int. Cl.: A61K 8/39, A61Q 1/14, A61Q 19/10, A61K 8/92, A61K 8/02, A61K 8/06

(54) **W/O-EMULSION ZUR TRÄNKUNG VON VLIESSTOFF**
W/O EMULSION FOR IMPREGNATING NONWOVEN FABRIC
ÉMULSION EAU-DANS-HUILE POUR L'IMPRÉGNATION DE NON-TISSÉ

(30) Priorität: 17.11.2017 DE 102017127199
(43) Veröffentlichungstag der Anmeldung: 23.09.2020
(73) Patentinhaber: Dr. Schumacher GmbH, 34323 Malsfeld (DE)
(72) Erfinder: BALLEZ, Mike, 34323 Malsfeld (DE); FUSS, Julia, 34323 Malsfeld (DE); NIELSEN, Jens, 34323 Malsfeld (DE)
(74) Vertreter: Sandvoß, Stefanie
(86) Internationale Anmeldenummer: PCT/DE2018/100937
(87) Internationale Veröffentlichungsnummer: WO 2019/096355

(56) Entgegenhaltungen:
- EP-A1- 2 092 929
- US-A1- 2010 242 796
- RIGANO L ET AL: "Olive oil- derived polyfunctional vehicles", SOFW JOURNAL, VERLAG FUER CHEMISCHE INDUSTRIE H. ZIOLKOWSKY GMBH, DE, Bd. 135, Nr. 6, 1. Juni 2009 (2009-06-01), Seiten 20-28, XP009169332, ISSN: 0942-7694
- J MEYER ET AL: "A Novel PEG-free Emulsifier Designed for Formulating W/O Lotions with a Light Skin Feel International Journal for Applied Science - Personal Care - Detergents - Specialties O IS A Novel PEG-free Emulsifier Designed for Formulating W/O Lotions with a Light Skin Feel", INTERNATIONAL JOURNAL FOR APPLIED SCIENCE, 1. November 2005 (2005-11-01), Seiten 20-26, XP055208714,
- "Emulsifiers for Skin Care Applications", , 1. März 2008 (2008-03-01), Seiten 1-20, XP055135519, Gefunden im Internet: URL:http://www.finecon.sk/userfiles/file/b roschuere_pc_3.pdf [gefunden am 2014-08-20]

## Beschreibung

Feuchttücher enthalten bekanntermaßen Vliesstoffe, die mit einer in der Regel flüssigen Zubereitung getränkt sind, die u.a. einen reinigenden und/oder pflegenden Inhaltsstoff enthält. Feuchttücher finden mittlerweile in vielen Bereichen der persönlichen Pflege und vor allem Babypflege Anwendung und erfreuen sich sehr hoher Beliebtheit, da sie die Möglichkeit einer schnellen und bequemen Reinigung bzw. Pflege bieten, ohne dass dafür zusätzliches Wasser erforderlich ist. In der Regel zu handlichen Einheiten von ca. 10 bis 100 Tüchern verpackt, zeichnen sich Feuchttücher darüber hinaus auch durch ihre Portabilität aus und sind vor allem auch aufgrund dieser Eigenschaft aus unserer immer mobiler werdenden Gesellschaft nicht mehr wegzudenken.

In der Regel sind die in Feuchttüchern enthaltenen Vliesstoffe mit Öl-in-Wasser-Emulsionen (O/W-Emulsionen) getränkt, bei denen die Ölphase dispers in der kontinuierlichen wässrigen Phase vorliegt. Einen größeren Pflegeeffekt als O/W-Emulsionen haben allerdings Wasserin-ÖI-Emulsionen (W/O-Emulsionen), bei denen die Ölphase die kontinuierliche Phase ist, in der die wässrige Phase dispers vorliegt. Ein weiterer Vorteil von W/O-Emulsionen gegenüber O/W-Emulsionen liegt darüber hinaus darin, dass in der wässrigen Phase enthaltene Wirkstoffe durch die umgebende Ölphase besser vor Oxidation geschützt sind.

Mit einer W/O-Emulsion getränkte Vliesstoffe als Feuchttücher wären daher erstrebenswert, insbesondere um einen größeren Pflegeeffekt bei der Anwendung der Feuchttücher zu erzielen.

W/O-Emulsionen sind allerdings aufgrund der im Vergleich zu O/W-Emulsionen erhöhten Viskosität nur schwer auf einen Vliesstoff aufzubringen. Weiterhin besteht insbesondere aufgrund der großen Oberfläche von Feuchttüchern zudem eine erhöhte Schwierigkeit darin, die Stabilität von auf einen Vliesstoff aufgebrachten W/O-Emulsionen über einen hinreichend langen Zeitraum zu gewährleisten.

Aus dem Stand der Technik bekannte Feuchttücher enthalten daher als Tränkungsmittel O/W-Emulsionen. Beispielhaft sei die EP 1 171 094 B1 erwähnt, die ein Feuchttuch beschreibt, dessen Vliesstoff mit einer Öl-in-Wasser-Emulsion getränkt ist, die Sterin oder ein Sterin-Derivat sowie einen Feuchtigkeitsspender enthält. Neben Wasser und optional Alkohol enthält das Tränkungsmittel zudem auch bis zu 30 Gew.-% natürliche Fette oder Öle.

Die WO 2013/120829 A1 beschreibt dünnflüssige W/O-Emulsionen, die spreitfähige Öle mit Ölphasen von 10 % bis 60 % enthalten und durch mindestens zwei W/O-Emulgatoren stabilisiert sind. Eine Tränkung von Vliesstoff mit den W/O-Emulsionen wird nicht beschrieben.

Die US 2011/0020258 A1 beschreibt eine dünnflüssige W/O-Emulsion zur Pflege der Haut und zur Makeup-Entfernung, die als Stabilisatoren einen lipophilen Emulgator und ein durch Modifizierung hydrophobisiertes Polysaccharid enthält. Eine Tränkung von Vliesstoff mit der W/O-Emulsion wird ebenfalls nicht beschrieben.

Die EP 2 921 159 A1 beschreibt eine W/O-Emulsion zur Entfernung wasserfesten Make-ups, die eine Ölkomponente mit einer Polarität von mindestens 20 mN/m, Wasser, einen lipophilen Emulgator sowie optional Antioxidantienten und Emollients enthält.

Die Druckschrift "Technical Information ISOLAN GPS", Evonik Nutrition & Care GmbH, beschreibt die Eignung von Polyglyceryl-4 Diisostearate/Polyhydroxystearate/Sebacate als Emulgator für W/O-Emulsionen mit geringer Viskosität, die jedoch nicht dazu geeignet sind, als Tränkungsmittel für Vliesstoff verwendet zu werden.

Die EP 2 092 929 A1 beschreibt eine W/O-Emulsion mit einem Lichtschutzfaktor ≥ 50, die gemäß Beispiel 1 Caprylic / Capric Triglyceride, Emollients, Wasser, Magnesiumsulfat, ein Antioxidans sowie Polyglyceryl-4 Diisostearate/Polyhydroxystearate/Sebacate als Emulgator enthält.

### Aufgabe der Erfindung

Es ist die Aufgabe der vorliegenden Erfindung, eine alternative W/O-Emulsion bzw. ein alternatives Feuchttuch bereitzustellen, wobei die W/O-Emulsion zur Tränkung von Vliesstoff geeignet und als Tränkungsmittel in dem Feuchttuch enthalten ist. Vorzugsweise soll die W/O-Emulsion dabei ein kostengünstigeres Tränkungsmittel sein als herkömmliche wasserfreie und ölhaltige Tränkungsmittel. Bevorzugt soll das Feuchttuch auch bei geringer Tränkmenge eine gute Reinigungsleistung bzw. einen guten Pflegeeffekt bieten.

### Allgemeine Beschreibung der Erfindung

Die Erfindung löst diese Aufgabe mit den Merkmalen der Ansprüche und insbesondere mit einer
W/O-Emulsion zur Tränkung von Vliesstoff, die
- 10 - 60 Gew.-% Pflanzenöl,
- 0,1 - 25 Gew.-% Emollients,
- 10 - 50 Gew.-% Wasser,
- einen lipophilen Emulgator,
- ein anorganisches Salz, und
- zumindest ein Antioxidans
enthält oder daraus besteht, wobei der Emulgator Polyglyceryl-4-Diisostearate/Polyhydroxystearate/ Sebacate ist. Die Viskosität der Emulsion beträgt vorzugsweise 250 - 600 mPa·s.

Alle im Zusammenhang mit der vorliegenden Erfindung ermittelten Viskositäten wurden mit einem Rotationsrheometer, Typ R180, DIN-Messsystem 33 gemäß ISO 53019 bei einer Temperatur von 293,15 K und mit einer Umdrehungszahl von 500 r/min bestimmt. Entsprechend beziehen sich auch alle in der vorliegenden Anmeldung enthaltenen Viskositätsangaben auf diese Messparameter.

Wie eingangs bereits erwähnt, ist es bei Feuchttüchern aufgrund ihrer großen Oberfläche besonders anspruchsvoll, die Stabilität der als Tränkungsmittel verwendeten Emulsionen zu gewährleisten und somit ein langzeitstabiles Endprodukt bereitzustellen. Dies trifft insbesondere bei W/O-Emulsionen zu, die naturgemäß eine höhere Viskosität aufweisen als O/W-Emulsion.

Während die Viskosität herkömmlicher W/O-Emulsionen beispielsweise bei ca. 800 mPa·s liegt, beträgt die Viskosität der erfindungsgemäßen W/O-Emulsion nur 250-600 mPa·s, beispielsweise 300-500 mPa·s oder 300-350 mPa·s. Gemäß einer bevorzugten Ausführungsform beträgt die Viskosität der erfindungsgemäßen W/O-Emulsion < 500 mPa·s, insbesondere 250-450 mPa·s, und gemäß einer besonders bevorzugten Ausführungsform beträgt die Viskosität der erfindungsgemäßen W/O-Emulsion < 450 mPa·s, insbesondere 250-400 mPa·s oder 250-350 mPa·s.

Für den Fachmann hat sich überraschend gezeigt, dass die erfindungsgemäße dünnflüssige W/O-Emulsion auf Basis von Planzenöl zur Verwendung in Feuchttüchern bzw. als Tränkungsmittel für den in Feuchttüchern verwendeten Vliesstoff geeignet ist. Dies wird insbesondere auf den hohen Anteil an pflanzlichen Ölen und auf den Emulgator Polyglyceryl-4 Diisostearate/Polyhydroxystearate/Sebacate sowie auf das Verhältnis von Ölphase zu Emulgator zurückgeführt.

Obwohl der in der erfindungsgemäßen W/O-Emulsion enthaltene Emulgator bereits aus der eingangs erwähnten Schrift "Technical Information ISOLAN GPS" bekannt ist, so sind die den Emulgator enthaltenden dort offenbarten Rezepturen nicht zur Tränkung von Vliesstoff geeignet, da selbst die zwei dünnflüssigsten Zusammensetzungen bereits eine Viskosität von > 700 mPa·s bzw. sogar > 900 mPa·s (gemessen mit einem Rotationsrheometer, Typ R180, DIN-Messsystem 33 gemäß ISO 53019 bei einer Temperatur von 293,15 K und mit einer Umdrehungszahl von 500 r/min) aufweisen und daher nicht niedrigviskos genug sind, um als Tränkungsmittel gleichmäßig auf einen Vliesstoff aufgebracht werden zu können. Weiterhin benötigen die aus dieser Schrift bekannten Emulsionen zusätzlich zu dem Emulgator Polyglyceryl-4-Diisostearate/Polyhydroxystearate/ Sebacate noch weitere konsistenzgebende Stabilisatoren, z.B. mikrokristallines Wachs oder Zinkstearat, insbesondere bei einer kalt/kalt Herstellung. Im Gegensatz dazu ist bei der erfindungsgemäßen Emulsion aufgrund der abweichenden Rahmenrezeptur mit besonders hohem Pflanzenölanteil kein weiterer organischer bzw. konsistenzgebender Stabilisator erforderlich und die erfindungsgemäße Emulsion enthält Polyglyceryl-4-Diisostearate/Polyhydroxystearate/ Sebacate bevorzugt als alleinigen Emulgator.

Der in der erfindungsgemäßen W/O-Emulsion enthaltene Ölanteil von 10 - 60 Gew.-%, bevorzugt von 20 - 50 Gew.-% und besonders bevorzugt von 25 - 45 Gew.-%, kann gemäß einer Ausführungsform durch nur ein Pflanzenöl gebildet werden, oder gemäß einer alternativen Ausführungsform durch mehrere verschiedene Pflanzenöle. Bevorzugt ist das Pflanzenöl bzw. sind die Pflanzenöle dabei ausgewählt aus der Gruppe, die Sonnenblumenöl, Rapsöl, Mandelöl, Jojobaöl, Avocadoöl, Kokosöl und Mischungen aus zwei oder mehr dieser Öle umfasst.

In der erfindungsgemäßen W/O-Emulsion sind weiterhin 0,1 - 25 Gew.-%, bevorzugter 5 - 20 Gew.-% und besonders bevorzugt 10 - 15 Gew.-% Emollients enthalten. Unter Emollients werden für die Zwecke der Erfindung ganz allgemein Verbindungen verstanden, die der Haut Geschmeidigkeit verleihen, z.B. Lipide, insbesondere mit einer Kettenlänge von ca. 14 bis 16 Kohlenstoffatomen.

Optional enthält die erfindungsgemäße W/O-Emulsion auch ein Esteröl oder mehrere Esteröle als Emollients, wobei der Gesamtanteil an Öl in der Emulsion bevorzugt 60 Gew.-% nicht überschreitet. Insbesondere können Isopropylmyristat, Isopropylpalmitat und/oder Isononanoate als Esteröl in der W/O-Emulsion enthalten sein.

Alternativ oder zusätzlich zu Esteröl bzw. Esterölen enthält die erfindungsgemäße W/O-Emulsion auch einen oder mehrere Dialkylether als Emollients, insbesondere Dicaprylyl Ether.

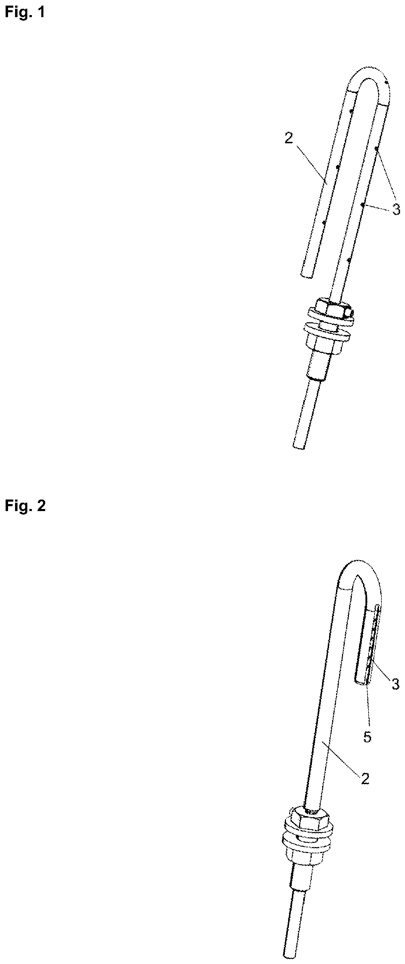

Der Emulgator Polyglyceryl-4 Diisostearate/Polyhydroxystearate/Sebacate weist die in Abbildung 1 gezeigte Strukturformel auf, in der PHS einen Polyhydroxystearat-Rest und IS einen Isostearat-Rest bezeichnet:

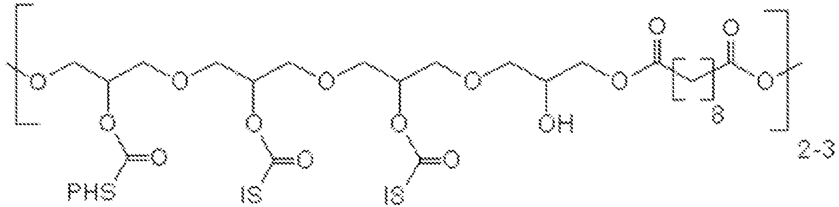

### Abbildung 1: Strukturformel des Emulgators Polyglyceryl-4 Diisostearate/Polyhydroxystearate/ Sebacate

Im Gegensatz zu aus dem Stand der Technik bekannten W/O-Emulsionen, die mehr als einen Emulgator enthalten, enthält die erfindungsgemäße W/O-Emulsion vorzugsweise ausschließlich die Verbindung Polyglyceryl-4 Diisostearate/Polyhydroxystearate/Sebacate als Emulgator.

Gemäß einer Ausführungsform ist der Emulgator Polyglyceryl-4 Diisostearate/Polyhydroxystearate/Sebacate in einem Verhältnis von 1:10 bis 1:20 zur Ölphase in der W/O-Emulsion enthalten, bevorzugter in einem Verhältnis von 1:12 bis 1:18 zur Ölphase, und besonders bevorzugt in einem Verhältnis von 1:14 bis 1:17 zur Ölphase.

Erfindungsgemäß sind in der W/O-Emulsion zudem 10 - 50 Gew.-% Wasser, vorzugsweise entionisiertes Wasser, enthalten. Die Wasserphase enthält dabei bevorzugt zumindest einen Wirkstoff, beispielsweise Glycerin, Urea, Panthenol, ein wasserlösliches Vitamin, z.B. Vitamin C, und/oder ein Pflanzenextrakt, z.B. Allantoin und/oder Aloe Vera.

Als Stabilisator enthält die erfindungsgemäße Emulsion ein anorganisches Salz, insbesondere ein Alkali- oder Erdalkalisalz. Bevorzugte Salze sind dabei Sulfate, insbesondere Natriumsulfat, Kaliumsulfat, Magnesiumsulfat oder Calciumsulfat. Gemäß einer bevorzugten Ausführungsform ist das anorganische Salz dabei in einem Verhältnis von 1:20 bis 1:40 zur Wasserphase und insbesondere in einem Verhältnis von 1:30 bis 1:35 zur Wasserphase in der erfindungsgemäßen W/O-Emulsion enthalten.

Ein weiterer Inhaltsstoff der Emulsion ist auch ein Antioxidans. Dieses kann beispielsweise ausgewählt sein aus der Gruppe, die Butylhydroxytoluol, Rosmarinextrakt, Tocopherolacetat und Isomere des Tocopherols umfasst. Optional sind mehrere Antioxidantien in der Emulsion enthalten, die ebenfalls aus der vorgenannten Gruppe ausgewählt sein können. Unabhängig davon, ob die W/O-Emulsion ein Antioxidans oder mehrere Antioxidantien enthält, beträgt der Gewichtsanteil an Antioxidans bevorzugt 0,01 - 0,025 Gew.-% pro Gew.-% Ölphase.

Vorteilhafterweise ist die erfindungsgemäße W/O-Emulsion, insbesondere aufgrund des Wasseranteils von bis zu 50 Gew.-%, kostengünstiger als herkömmliche wasserfreie Tränkungsmittel auf Ölbasis.

Die Erfindung betrifft außerdem auch ein Verfahren zur Herstellung einer W/O-Emulsion, die
- 10 - 60 Gew.-% Pflanzenöl,
- 0,1 - 25 Gew.-% Emollients,
- 10 - 50 Gew.-% Wasser,
- einen lipophilen Emulgator,
- ein anorganisches Salz, und
- zumindest ein Antioxidans
enthält oder daraus besteht, wobei der Emulgator Polyglyceryl-4 Diisostearate/Polyhydroxystearate/Sebacate ist und die Viskosität der Emulsion 250 - 600 mPa·s beträgt.

In dem erfindungsgemäßen Verfahren wird eine wässrige Phase, die Wasser, ein anorganisches Salz und zumindest ein Antioxidans sowie optional zumindest einen Wirkstoff enthält, unter Rühren in eine den Emulgator Polyglyceryl-4 Diisostearate/Polyhydroxystearate/Sebacate enthaltende Ölphase gegeben.

Die Herstellung der W/O-Emulsion einschließlich des Einrührens der wässrigen Phase in die Ölphase erfolgt dabei vorzugsweise vollständig bei Raumtemperatur, d.h. ohne Erhitzen. Dies ist möglich, da die erfindungsgemäße Emulsion keine festen Inhaltsstoffe enthält, die durch Hitzezufuhr verflüssigt werden müssen.

Im Übrigen weisen alle in dem erfindungsgemäßen Verfahren verwendeten Inhaltsstoffe, d.h. das Pflanzenöl, die Emollients, das Wasser, der lipophile Emulgator, das anorganische Salz und das zumindest eine Antioxidans dieselbe Beschaffenheit auf wie im Vorstehenden mit Bezug auf die erfindungsgemäße W/O-Emulsion beschrieben. Dies gilt insbesondere auch für die Art der Inhaltsstoffe, z.B. die Art des Pflanzenöls, des Salzes und des Antioxidans, sowie für deren Gewichtsanteile.

Optional enthält die in dem erfindungsgemäßen Verfahren eingesetzte Ölphase neben Pflanzenöl auch Esteröl als Emollient, wobei der Gesamtanteil an eingesetztem Pflanzenöl und eingesetztem Esteröl in der Emulsion max. 60 Gew.-% beträgt. Insbesondere können Isopropylmyristat, Isopropylpalmitat und/oder Isononanoate als Esteröl in der Herstellung der W/O-Emulsion eingesetzt werden.

Die Erfindung betrifft weiterhin auch ein Feuchttuch, das einen Vliesstoff umfasst, der mit einer erfindungsgemäßen W/O-Emulsion getränkt ist, d.h. mit einer W/O-Emulsion, die
- 10 - 60 Gew.-% Pflanzenöl,
- 0,1 - 25 Gew.-% Emollients,
- 10 - 50 Gew.-% Wasser,
- einen lipophilen Emulgator,
- ein anorganisches Salz, und
- zumindest ein Antioxidans
enthält oder daraus besteht, wobei der Emulgator Polyglyceryl-4 Diisostearate/Polyhydroxystearate/ Sebacate ist und die Viskosität der Emulsion 250 - 600 mPa·s beträgt.

Zur Herstellung des erfindungsgemäßen Feuchttuchs wird die W/O-Emulsion vorzugsweise mit einer speziellen Düseneinheit auf den Vliesstoff aufgebracht. Diese umfasst mehrere Auslassröhrchen, die jeweils mehrere Auslassöffnungen aufweisen. Die Auslassöffnungen sind dabei in einer Vertiefung angeordnet, die in Längsrichtung des Auslassröhrchens angeordnet ist und sich über einen Teil der Länge des Auslassröhrchens oder über dessen gesamte Länge erstreckt. Beispielsweise kann die Vertiefung in die Auslassröhrchen hineingefräst sein.

Dadurch, dass die Auslassöffnungen jeweils in einer in Längsrichtung der Auslassröhrchen angeordneten Vertiefung angeordnet sind, sammelt sich die aus den Auslassöffnungen austretende W/O-Emulsion zunächst in dieser Vertiefung an und wird anschließend filmartig auf den Vliesstoff aufgebracht, um diesen zu tränken. Auf diese Weise ist eine gleichmäßige Tränkung des Vliesstoffes mit der im Vergleich zu O/W-Emulsionen höherviskosen Emulsion möglich. Eine gleichmäßige Tränkung mittels einer aus dem Stand der Technik bekannten Düseneinheit ohne entsprechende Vertiefung im Bereich der Auslassöffnungen, in der sich die Emulsion ansammeln könnte, ist aufgrund der höheren Viskosität der Emulsion nicht möglich, sondern würde nur zu einer punktuellen bzw. ungleichmäßigen Tränkung führen.

Gemäß einer bevorzugten Ausführungsform umfasst die Düseneinheit, mittels derer die W/O-Emulsion auf den Vliesstoff aufgebracht wird, wie auch aus dem Stand der Technik bekannt mehrere Auslassröhrchen, die jedoch eine höhere Anzahl Auslassöffnungen aufweisen, als dies aus dem Stand der Technik bekannt ist. Diese erhöhte Anzahl an Auslassöffnungen dient insbesondere dazu, die in Längsrichtung der Auslassröhrchen angeordneten Vertiefungen für die Tränkung möglichst effektiv mit Emulsion zu füllen. Die Vertiefung fungiert dabei als Art Reservoir für die aufzubringende W/O-Emulsion, mit dem der Vliesstoff zur Tränkung kontaktiert wird.

Gemäß einer Ausführungsform besteht der in dem erfindungsgemäßen Feuchttuch enthaltene Vliesstoff aus Viskosefasern und/oder Lyocell-Fasern und/oder PET-Fasern, d.h. er kann entweder zu 100 % aus Viskosefasern, Lyocell-Fasern oder PET-Fasern bestehen, oder aus Mischfasern dieser bestehen.

Optional enthält der Vliesstoff auch Mikrofasern und/oder Baumwoll-Fasern, beispielsweise zu einem Anteil von jeweils bis zu 10 Gew.-%, insbesondere von 0,1 - 5,0 Gew.-%.

Gemäß einer bevorzugten Ausführungsform besteht der in dem erfindungsgemäßen Feuchttuch enthaltene Vliesstoff aus Viskosefasern und/oder Lyocell-Fasern und/oder PET-Fasern und zusätzlich aus 0,1 - 5,0 Gew.-% Mikrofasern und/oder 0,1 - 5,0 Gew.-% Baumwoll-Fasern.

Besonders bevorzugt umfasst oder besteht der Vliesstoff eines erfindungsgemäßen Feuchttuches aus 30 - 70 Gew.-% Viskosefasern und 30 - 70 Gew.-% PET-Fasern sowie optional 0,1 - 5,0 Gew.-% Mikrofasern und/oder 0,1 - 5,0 Gew.-% Baumwoll-Fasern.

In Ausführungsformen, in denen der Vliesstoff weder Lyocell-Fasern, noch Mikrofasern oder Baumwoll-Fasern enthält, sondern ausschließlich aus Viskosefasern und PET-Fasern besteht, kann der Anteil von Viskosefasern zu PET-Fasern stufenlos variieren, beispielsweise von 1 Gew.-% Viskosefasern / 99 Gew.-% PET-Fasern zu 99 Gew.-% Viskosefasern / 1 Gew.-% PET-Fasern.

Da es sich bei dem Tränkungsmittel des erfindungsgemäßen Feuchttuchs um die im Voranstehenden beschriebene erfindungsgemäße W/O-Emulsion handelt, gelten alle im Zusammenhang mit der Beschreibung der erfindungsgemäßen Emulsion offenbarten Merkmale, insbesondere die Konkretisierung hinsichtlich der Inhaltsstoffe sowie deren Gewichtsanteile, auch für die als Tränkungsmittel für das erfindungsgemäße Feuchttuch verwendete W/O-Emulsion.

Insbesondere durch seine im Vergleich zu vorbekannten W/O-Emulsionen geringe Viskosität von 250 - 600 mPa·s, bevorzugt 300 - 500 mPa·s und besonders bevorzugt 300 - 350 mPa·s, ist das in dem erfindungsgemäßen Feuchttuch auf einen Vliesstoff aufgebrachte Tränkungsmittel stabil, insbesondere oxidationsstabil. Dabei enthält das Tränkungsmittel vorzugsweise ausschließlich den Emulgator Polyglyceryl-4 Diisostearate/Polyhydroxystearate/Sebacate und keinen weiteren Emulgator. Überdies enthält das Tränkungsmittel auch keine Mineralöle.

Es hat sich überraschend gezeigt, dass erfindungsgemäße Feuchttücher auch bei einer geringen Tränkmenge, beispielsweise von max. 1,0 g Tränkungsmittel pro Gramm Vliesstoff, eine besonders gute Reinigungsleistung bzw. einen besonders guten Pflegeeffekt erzielen. Bevorzugt sind sie zudem kostengünstiger herstellbar als aus dem Stand der Technik bekannte wasserfreie Feuchttücher auf Ölbasis.

Das Tränkungsmittel ist überdies zur Aufnahme verschiedener Wirkstoffe geeignet bzw. kann verschiedene Wirkstoffe enthalten. Je nach Beschaffenheit des in dem Tränkungsmittel enthaltenen Wirkstoffes bzw. der in dem Tränkungsmittel enthaltenen Wirkstoffe eignet sich das erfindungsgemäße Feuchttuch beispielsweise zur Gesichts- und/oder Körperreinigung, als Anti-Aging-Mittel, als Anti-Pollution-Mittel, als Gesichtsmaske, zur Make-up-Entfernung, als Toilettenpapier und/oder zur Babyreinigung und/oder -pflege. Entsprechend betrifft die Erfindung auch die Verwendung eines erfindungsgemäßen Feuchttuches zur Gesichtsund/oder Körperreinigung, als Anti-Aging-Mittel, als Anti-Pollution-Mittel, als Gesichtsmaske, zur Make-up-Entfernung, als Toilettenpapier und/oder zur Babyreinigung und/oder -pflege.

### Genaue Beschreibung der Erfindung

Die Erfindung wird nun genauer anhand von Ausführungsbeispielen und mit Bezug zu den Figuren beschrieben, die schematisch
- in der Figur 1 ein aus dem Stand der Technik bekanntes Auslassröhrchen zum Aufbringen von Tränkungsmittel auf Vliesstoff,
- in der Figur 2 ein in einem erfindungsgemäßen Verfahren zur Herstellung eines Feuchttuchs verwendetes Auslassröhrchen zum Aufbringen der Emulsion auf Vliesstoff,
- in der Figur 3 ein weiteres in einem erfindungsgemäßen Verfahren zur Herstellung eines Feuchttuchs verwendetes Auslassröhrchen zum Aufbringen der Emulsion auf Vliesstoff, und
- in der Figur 4 eine in einem erfindungsgemäßen Verfahren zur Herstellung eines Feuchttuchs eingesetzte Düseneinheit zeigen.

Zunächst wird die Herstellung erfindungsgemäßer W/O-Emulsionen anhand der nachstehenden zwei Beispiele beschrieben, die jeweils Rahmenrezepturen für erfindungsgemäße W/O-Emulsionen enthalten. Die Gewichtsanteile der einzelnen Inhaltsstoffe an den Emulsionen können dabei jeweils in dem angegebenen Rahmen variieren.

### Beispiel 1: Herstellung einer erfindungsgemäßen W/O-Emulsion auf Basis von Rapsöl

Die Tabelle 1 enthält eine Rahmenrezeptur für eine erfindungsgemäße W/O-Emulsion auf Basis von Rapsöl.

Alle mit "Phase A" gekennzeichneten Inhaltsstoffe sind Bestandteile der wässrigen Phase, alle mit "Phase B" gekennzeichneten Inhaltsstoffe sind Bestandteile der Ölphase.

**Tabelle 1: Rahmenrezeptur einer W/O-Emulsion auf Basis von Rapsöl**

| **Inhaltsstoffe** | **Konzentration (Gew.-%)** | **Phase** |
|---|---|---|
| Wasser | Ad 100,00 | A |
| Rapsöl | 25,00 - 50,00 | B |
| Rosmarinextrakt | 0,01 - 0,10 | B |
| Magnesiumsulfat | 1,50 | A |
| Polyglyceryl-4 Diisostearate/Polyhydroxystearate/Sebacate | 1,50 - 3,00 | B |
| Glycerin | 1,00 - 5,00 | A |
| Zitronensäure | 0,20 | A |
| Natriumbenzoat | 0,50 | A |
| Parfum | 0,01 - 0,40 | B |

Für die Herstellung der wässrigen Phase wird zunächst bei Raumtemperatur die erforderliche Menge an Wasser in einem Gefäß vorgelegt. Anschließend werden ebenfalls bei Raumtemperatur Magnesiumsulfat, Glycerin, Zitronensäure und Natriumbenzoat hinzugegeben und es wird bis zum Erhalt einer klaren Lösung gerührt.

Für die Herstellung der Ölphase wird Rapsöl bei Raumtemperatur in einem zweiten Gefäß vorgelegt. Nun werden Rosmarinextrakt, der Emulgator Polyglyceryl-4-Diisostearate/Polyhydroxy-stearate/Sebacate und Parfum hinzugefügt und es wird bis zum Erhalt einer klaren Ölphase gerührt.

Anschließend wird die wässrige Phase unter starkem Rühren und Homogenisieren zu der Ölphase gegeben, wodurch die erfindungsgemäße W/O-Emulsion erhalten wird.

Die Viskosität der erhaltenen W/O-Emulsion nach der in Tabelle 1 angegebenen Rahmenrezeptur liegt bei 350 - 500 mPa·s (bestimmt mit DIN-Messsystem 33 gemäß ISO 53019 bei einer Temperatur von 293,15 K und mit einer Umdrehungszahl von 500 r/min), wobei die Viskosität innerhalb dieses Rahmens durch die Intensität des Homogenisierens beeinflusst werden kann. Kurzes Homogenisieren führt dabei zu Viskositäten im unteren Bereich des angegebenen Rahmens, und längeres Homogenisieren führt zu Viskositäten im oberen Bereich des angegebenen Rahmens.

### Beispiel 2: Herstellung einer erfindungsgemäßen W/O-Emulsion auf Basis von Sonnenblumenöl

Die Tabelle 2 enthält eine Rahmenrezeptur für eine erfindungsgemäße W/O-Emulsion auf Basis von Sonnenblumenöl.

Alle mit "Phase A" gekennzeichneten Inhaltsstoffe sind Bestandteile der wässrigen Phase, alle mit "Phase B" gekennzeichneten Inhaltsstoffe sind Bestandteile der Ölphase.

**Tabelle 2: Rahmenrezeptur einer W/O-Emulsion auf Basis von Sonnenblumenöl**

| **Inhaltsstoffe** | **Konzentration (Gew.-%)** | **Phase** |
|---|---|---|
| Wasser | Ad 100,00 | A |
| Sonnenblumenöl | 15,00 - 30,00 | B |
| Rosmarinextrakt | 0,01 - 0,10 | B |
| Magnesiumsulfat | 0,25 - 0,75 | A |
| Polyglyceryl-4 Diisostearate/Polyhydroxystearate/Sebacate | 0,75 - 2,00 | B |
| Ethylhexylstearat | 5,00 - 10,00 | B |
| Glycerin | 1,00 - 5,00 | A |
| Isopropylmyristat | 5,00 - 10,00 | B |
| Zitronensäure | 0,15 | A |
| Mandelöl | 0,50 - 5,00 | B |
| Natriumbenzoat | 0,50 | A |
| Parfum | 0,01 - 0,40 | B |

Für die Herstellung der wässrigen Phase wird zunächst bei Raumtemperatur die erforderliche Menge an Wasser in einem Gefäß vorgelegt. Anschließend werden ebenfalls bei Raumtemperatur Magnesiumsulfat, Glycerin, Zitronensäure und Natriumbenzoat hinzugegeben und es wird bis zum Erhalt einer klaren Lösung gerührt.

Für die Herstellung der Ölphase wird Sonnenblumenöl bei Raumtemperatur in einem zweiten Gefäß vorgelegt. Nun werden Rosmarinextrakt, der Emulgator Polyglyceryl-4-Diiso-stearate/Polyhydroxystearate/Sebacate, Ethylhexylstearat, Isopropylmyristat, Mandelöl und Parfum hinzugefügt und es wird bis zum Erhalt einer klaren Ölphase gerührt.

Anschließend wird die wässrige Phase unter starkem Rühren und Homogenisieren zu der Ölphase gegeben, wodurch die erfindungsgemäße W/O-Emulsion erhalten wird.

Die Viskosität der erhaltenen W/O-Emulsion nach der in Tabelle 2 angegebenen Rahmenrezeptur liegt bei 330 - 400 mPa·s (bestimmt mit DIN-Messsystem 33 gemäß ISO 53019 bei einer Temperatur von 293,15 K und mit einer Umdrehungszahl von 500 r/min), wobei die Viskosität innerhalb dieses Rahmens durch die Intensität des Homogenisierens beeinflusst werden kann. Kurzes Homogenisieren führt dabei zu Viskositäten im unteren Bereich des angegebenen Rahmens, und längeres Homogenisieren führt zu Viskositäten im oberen Bereich des angegebenen Rahmens.

### Beispiel 3: Herstellung einer weiteren erfindungsgemäßen W/O-Emulsion auf Basis von Rapsöl

Die Tabelle 3 enthält eine Rahmenrezeptur für eine erfindungsgemäße W/O-Emulsion auf Basis von Rapsöl.

Alle mit "Phase A" gekennzeichneten Inhaltsstoffe sind Bestandteile der wässrigen Phase, alle mit "Phase B" gekennzeichneten Inhaltsstoffe sind Bestandteile der Ölphase.

**Tabelle 3: Rahmenrezeptur einer W/O-Emulsion auf Basis von Rapsöl**

| **Inhaltsstoffe** | **Konzentration (Gew.-%)** | **Phase** |
|---|---|---|
| Wasser | Ad 100,00 | A |
| Rapsöl | 15,00 - 30,00 | B |
| Rosmarinextrakt | 0,01 - 0,10 | B |
| Magnesiumsulfat | 0,25 - 0,75 | A |
| Polyglyceryl-4 Diisostearate/Polyhydroxystearate/Sebacate | 0,75 - 2,00 | B |
| Ethylhexylstearat | 5,00 - 10,00 | B |
| Glycerin | 1,00 - 5,00 | A |
| Dicaprylyl Ether | 5,00 - 10,00 | B |
| Zitronensäure | 0,15 | A |
| Kokosöl | 0,25 - 5,00 | B |
| Natriumbenzoat | 0,50 | A |
| Parfum | 0,01 - 0,40 | B |
| Olivenöl | 0,25 - 5,00 | B |

Für die Herstellung der wässrigen Phase wird zunächst bei Raumtemperatur die erforderliche Menge an Wasser in einem Gefäß vorgelegt. Anschließend werden ebenfalls bei Raumtemperatur Magnesiumsulfat, Glycerin, Zitronensäure und Natriumbenzoat hinzugegeben und es wird bis zum Erhalt einer klaren Lösung gerührt.

Für die Herstellung der Ölphase wird Rapsöl bei Raumtemperatur in einem zweiten Gefäß vorgelegt. Nun werden Rosmarinextrakt, der Emulgator Polyglyceryl-4-Diisostearate/Polyhydroxy-stearate/Sebacate, Ethylhexylstearat, Dicaprylyl Ether, Kokosöl, Olivenöl und Parfum hinzugefügt und es wird bis zum Erhalt einer klaren Ölphase gerührt.

Anschließend wird die wässrige Phase unter starkem Rühren und Homogenisieren zu der Ölphase gegeben, wodurch die erfindungsgemäße W/O-Emulsion erhalten wird.

Die Viskosität der erhaltenen W/O-Emulsion nach der in Tabelle 3 angegebenen Rahmenrezeptur liegt bei 250 - 400 mPa·s (bestimmt mit DIN-Messsystem 33 gemäß ISO 53019 bei einer Temperatur von 293,15 K und mit einer Umdrehungszahl von 500 r/min), wobei die Viskosität innerhalb dieses Rahmens durch die Intensität des Homogenisierens beeinflusst werden kann. Kurzes Homogenisieren führt dabei zu Viskositäten im unteren Bereich des angegebenen Rahmens, und längeres Homogenisieren führt zu Viskositäten im oberen Bereich des angegebenen Rahmens.

Die Figur 1 zeigt ein aus dem Stand der Technik bekanntes Auslassröhrchen 2 zum Aufbringen von Tränkungsmittel auf Vliesstoff. Dieses ist in der gezeigten Ausführungsform gebogen und weist über seine gesamte Länge verteilt Auslassöffnungen 3 auf, durch welche Tränkungsmittel, das gemäß dem Stand der Technik in der Regel eine O/W-Emulsion ist, gleichmäßig auf Vliesstoff aufgebracht werden kann.

Erfindungsgemäße W/O-Emulsionen, z.B. nach Beispiel 1 oder nach Beispiel 2 hergestellt, weisen eine höhere Viskosität auf als O/W-Emulsionen. Würde man eine erfindungsgemäße Emulsion wie aus dem Stand der Technik bekannt mittels der in Fig. 1 gezeigten Auslassröhrchen **2** auf einen Vliesstoff aufzubringen versuchen, würde dies aufgrund der höheren Viskosität zu einer ungleichmäßigen bzw. punktuellen Tränkung des Vliesstoffes führen.

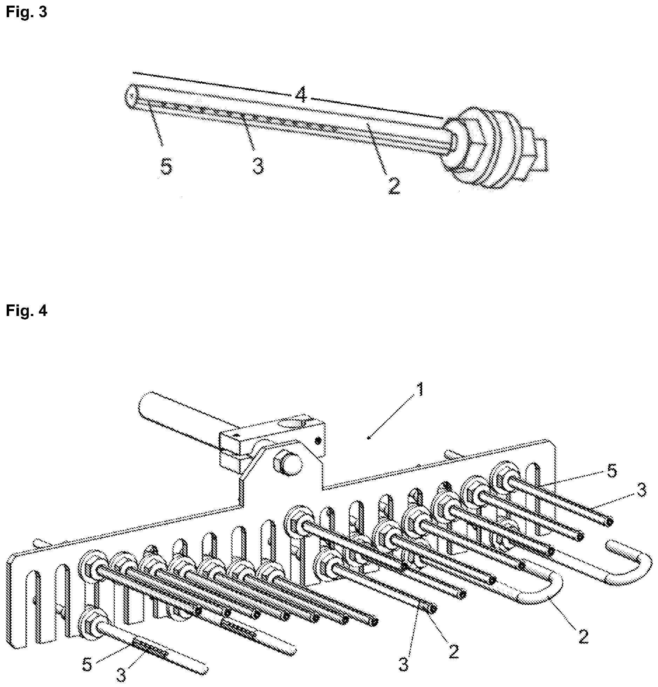

Zur Herstellung erfindungsgemäßer mit W/O-Emulsion getränkter Feuchttücher wird daher die schematisch in Fig. 4 gezeigte Düseneinheit **1** verwendet, die über mehrere der in Fig. 2 gezeigten gebogenen Auslassröhrchen **2** sowie mehrere der in der Fig. 3 gezeigten geraden Auslassröhrchen **2** verfügt. Sowohl die gebogenen Auslassröhrchen **2** als auch die geraden Auslassröhrchen **2** weisen dabei Auslassöffnungen **3** auf, die in einer sich in Längsrichtung der Auslassröhrchen **2** erstreckenden, z.B. eingefrästen, Vertiefung **5** angeordnet sind. Die Vertiefung **5** mit den darin angeordneten Auslassöffnungen **3** kann sich dabei über die gesamte Länge **4** der Auslassröhrchen **2** (wie in Figur 3 gezeigt) oder aber nur über einen Teil deren Länge (wie in Figur 2 gezeigt) erstrecken. Vorzugsweise weisen die Auslassröhrchen **2** dabei nur in der Vertiefung **5** angeordnete Auslassöffnungen **2** auf, sodass die aus den Auslassöffnungen **2** austretende W/O-Emulsion direkt in die Vertiefung **5** gelangt. Auf diese Weise füllt sich bei Austritt von Emulsion durch die Auslassöffnungen **3** die Vertiefung **5** wie ein Flüssigkeitsreservoir und kann bei Kontaktierung mit dem Vliesstoff gleichmäßig auf diesen aufgebracht werden.

Die in der vorstehenden Beschreibung sowie in den Ansprüchen offenbarten Merkmale der Erfindung können sowohl einzeln als auch in beliebigen Kombinationen für die Verwirklichung der Erfindung in ihren verschiedenen Ausführungsformen wesentlich sein.

## Patentansprüche

1. W/O-Emulsion zur Tränkung von Vliesstoff, enthaltend
- 10 - 60 Gew.-% Pflanzenöl,
- 0,1 - 25 Gew.-% Emollients,
- 10 - 50 Gew.-% Wasser,
- einen lipophilen Emulgator,
- ein anorganisches Salz,
- zumindest ein Antioxidans,
**dadurch gekennzeichnet, dass** der Emulgator Polyglyceryl-4 Diisostearate/Polyhydroxystearate/Sebacate ist und die Viskosität der Emulsion 250 - 600 mPa·s, bestimmt mit DIN-Messsystem 33 gemäß ISO 53019 bei einer Temperatur von 293,15 K und mit einer Umdrehungszahl von 500 r/min, beträgt.

2. W/O-Emulsion nach Anspruch 1, **dadurch gekennzeichnet, dass** das Pflanzenöl ausgewählt ist aus der Gruppe, die Sonnenblumenöl, Rapsöl, Mandelöl, Jojobaöl, Avocadoöl, Kokosöl und Mischungen dieser umfasst.

3. W/O-Emulsion nach einem der Ansprüche 1 oder 2, **dadurch gekennzeichnet, dass** der Emulgator im Verhältnis 1:10 bis 1:20 zur Ölphase enthalten ist.

4. W/O-Emulsion nach einem der voranstehenden Ansprüche, **dadurch gekennzeichnet, dass** die Emollients Esteröl und/oder Dialkylether enthalten.

5. W/O-Emulsion nach einem der voranstehenden Ansprüche, **dadurch gekennzeichnet, dass** der Gewichtsanteil an Antioxidans 0,01 - 0,025 Gew.-% pro Gew.-% Ölphase beträgt.

6. Verfahren zur Herstellung einer W/O-Emulsion nach einem der Ansprüche 1 bis 5, **dadurch gekennzeichnet, dass** eine wässrige Phase, die Wasser, ein anorganisches Salz und zumindest ein Antioxidans enthält, unter Rühren in eine den Emulgator Polyglyceryl-4 Diisostearate/Polyhydroxystearate/Sebacate enthaltende Ölphase gegeben wird.

7. Verfahren nach Anspruch 6, **dadurch gekennzeichnet, dass** die Herstellung der W/O-Emulsion vollständig bei Raumtemperatur erfolgt.

8. Feuchttuch umfassend einen Vliesstoff, **dadurch gekennzeichnet, dass** der Vliesstoff mit einer W/O-Emulsion nach einem der Ansprüche 1 bis 5 getränkt ist.

9. Verfahren zur Herstellung eines Feuchttuches nach Anspruch 8, **dadurch gekennzeichnet, dass** die W/O-Emulsion mittels einer mehrere Auslassröhrchen (2) aufweisenden Düseneinheit (1) auf den Vliesstoff aufgebracht wird, wobei jedes Auslassröhrchen (2) mehrere Auslassöffnungen (3) aufweist, die in einer in Längsrichtung des Auslassröhrchens (2) angeordneten und sich zumindest über einen Teil der Länge (4) des Auslassröhrchens (2) erstreckenden Vertiefung (5) angeordnet sind.

10. Verfahren nach Anspruch 9, **dadurch gekennzeichnet, dass** sich die W/O-Emulsion beim Austritt aus den in den Auslassröhrchen (2) angeordneten Auslassöffnungen (3) zunächst in der Vertiefung (5) ansammelt und anschließend auf den Vliesstoff aufgebracht wird.

## Claims

1. W/O emulsion for impregnating nonwoven, containing
- 10 - 60 % by weight of vegetable oil,
- 0.1 - 25 % by weight of emollients,
- 10 - 50 % by weight of water,
- a lipophilic emulsifier,
- an inorganic salt,
- at least one antioxidant,
**characterized in that** the emulsifier is polyglyceryl-4 diisostearate/polyhydroxystearate/sebacate and the viscosity of the emulsion is 250 - 600 mPa·s, determined using DIN measurement system 33 in accordance with ISO 53019 at a temperature of 293.15 K and at a rotation speed of 500 r/min.

2. W/O emulsion according to claim 1, **characterized in that** the vegetable oil is selected from the group comprising sunflower oil, rapeseed oil, almond oil, jojoba oil, avocado oil, coconut oil and mixtures thereof.

3. W/O emulsion according to one of the claims 1 or 2, **characterized in that** the emulsifier is contained in a ratio of 1:10 to 1:20 to the oil phase.

4. W/O emulsion according to one of the preceding claims, **characterized in that** the emollients contain ester oil and/or dialkyl ether.

5. W/O emulsion according to one of the preceding claims, **characterized in that** the proportion by weight of antioxidant is 0.01-0.025 % by weight per % by weight of oil phase.

6. Process for production of a W/O emulsion according to one of the claims 1 to 5, **characterized in that** an aqueous phase containing water, an inorganic salt and at least one antioxidant is added to an oil phase containing the emulsifier polyglyceryl-4 diisostearate/polyhydroxystearate/sebacate while stirring.

7. Process according to claim 6, **characterized in that** the production of the W/O emulsion takes place entirely at room temperature.

8. Wet wipe comprising a nonwoven, **characterized in that** the nonwoven is impregnated with a W/O emulsion according to one of the claims 1 to 5.

9. Method for production of a wet wipe according to claim 8, **characterized in that** the W/O emulsion is applied to the nonwoven using a nozzle unit (1) having a plurality of outlet tubes (2), each outlet tube (2) having a plurality of outlet openings (3) which are arranged in a recess (5) which is arranged in the longitudinal direction of the outlet tube (2) and extends at least over a portion of the length (4) of the outlet tube (2).

10. Method according to claim 9, **characterized in that** the W/O emulsion upon emerging from the outlet openings (3) arranged in the outlet tubes (2) initially collects within the recess (5) and is then applied to the nonwoven.

## Revendications

1. Émulsion E/H destinée à l'imprégnation de non-tissé, contenant
- 10 - 60 % en poids d'huile végétale,
- 0,1 - 25 % en poids d'émollients,
- 10 - 50 % en poids d'eau,
- un émulsifiant lipophile,
- un sel inorganique,
- au moins un antioxydant,
**caractérisée en ce que** l'émulsifiant est le polyglycéryl-4 diisostéarate/ polyhydroxystéarate/sébaçate et la viscosité de l'émulsion vaut 250 - 600 mPa.s, déterminée par le système de mesure DIN 33 selon ISO 53019 à une température de 293,15 K et à une vitesse de rotation de 500 r/min.

2. Émulsion E/H selon la revendication 1, **caractérisée en ce que** l'huile végétale est choisie dans le groupe qui comprend l'huile de tournesol, l'huile de colza, l'huile d'amande, l'huile de jojoba, l'huile d'avocat, l'huile de coprah et des mélanges de celles-ci.

3. Émulsion E/H selon l'une quelconque des revendications 1 ou 2, **caractérisée en ce que** l'émulsifiant est contenu en un rapport à la phase huileuse de 1:10 à 1:20.

4. Émulsion E/H selon l'une quelconque des revendications précédentes, **caractérisée en ce que** les émollients contiennent de l'huile à base d'esters et/ou un éther de dialkyle.

5. Émulsion E/H selon l'une quelconque des revendications précédentes, **caractérisée en ce que** la proportion en poids d'antioxydant vaut 0,01 - 0,025 % en poids par rapport au poids de la phase huileuse.

6. Procédé pour la préparation d'une émulsion E/H selon l'une quelconque des revendications 1 à 5, **caractérisée en ce qu'**on introduit sous agitation une phase aqueuse, qui contient de l'eau, un sel inorganique et au moins un antioxydant, dans une phase huileuse contenant l'émulsifiant polyglycéryl-4 diisostéarate/polyhydroxystéarate/sébaçate.

7. Procédé selon la revendication 6, **caractérisé en ce que** la préparation de l'émulsion E/H s'effectue entièrement à la température ambiante.

8. Lingette humide comprenant un non-tissé, **caractérisée en ce que** le non-tissé est imprégné avec une émulsion E/H selon l'une quelconque des revendications 1 à 5.

9. Procédé pour la fabrication d'une lingette humide selon la revendication 8, **caractérisé en ce que** l'émulsion E/H est appliquée sur le non-tissé au moyen d'une unité d'éjection (1) comportant plusieurs petits tubes d'éjection (2), chaque petit tube d'éjection (2) comportant plusieurs orifices d'éjection (3) qui sont disposés dans une cavité (5) disposée en direction longitudinale du petit tube d'éjection (2) et s'étendant au moins sur une partie de la longueur (4) du petit tube d'éjection (2).

10. Procédé selon la revendication 9, **caractérisé en ce que** l'émulsion E/H d'abord s'accumule dans la cavité (5) en sortant des orifices des orifices d'éjection (3) disposés dans les petits tubes d'éjection (2) et ensuite est appliquée sur le non-tissé.
